# EUROPEAN PATENT SPECIFICATION

(11) **EP 0 680 515 B1**
(45) Date of publication and mention of the grant of the patent: **26.08.1998**
(21) Application number: 94904295.6
(22) Date of filing: 21.01.1994
(51) Int. Cl.: C12Q 1/04, C12Q 1/48, C12Q 1/66

(54) **MICROBIOLOGICAL TEST METHOD AND REAGENTS**
MIKROBIOLOGISCHES TESTVERFAHREN UND REAGENZIEN
REACTIFS ET PROCEDE D'ESSAI MICROBIOLOGIQUE

(30) Priority: 21.01.1993 GB 9301118
(43) Date of publication of application: 08.11.1995
(73) Proprietor: Secretary of State for Defence in Her Britannic Majesty's Gov. of the United Kingdom of Great Britain and Northern Ireland, London SW1A 2HB (GB)
(72) Inventor: SQUIRRELL, David James, Salisbury, Wiltshire SP4 0JQ (GB)
(74) Representative: Beckham, Robert William
(86) International application number: GB9400118
(87) International publication number: WO9417202

(56) References cited:
- EP-A- 0 054 676
- EP-A- 0 238 352
- US-A- 3 933 592
- JOURNAL OF BIOCHEMICAL AND BIOPHYSICAL METHODS vol. 1, no. 3 , 1979 , AMSTERDAM, NL pages 163 - 169 SVEN E. BROLIN, ERIK BORGLUND AND AMBJÖRN A GREN 'Photokinetic Microassay of Adenylate Kinase Using the Firefly Luciferase Reaction' cited in the application
- CHEMICAL ABSTRACTS, vol. 96, no. 21, 24 May 1982, Columbus, Ohio, US; abstract no. 177160k, SCHIMZ, KARL LUDWIG ET AL. 'Determination of adenosine nucleotides with luciferin/luciferase from crude firefly lantern extract on a bioluminescence analyzer' page 350 ;
- Sigma Chemical Co.,(1992) Catalog of commercial products (page 45)

## Description

The present invention relates to a method for detecting microorganisms, to apparatus for carrying out the method and to test kits comprising essential reagents for carrying out the method.

All living organisms utilise adenosine triphosphate (ATP) as a source of chemical energy and it is known to assay this using the ATP driven luciferase/luciferin reaction. Light generated by this enzymic reaction can be measured using a luminometer and related to the amount of ATP present. The usefulness of ATP as an index of microbial numbers has been known since the mid 1960's (see ATP Luminescence Rapid Methods in Microbiology (1989) editor Stanley et al.; Blackwell Scientific Publications, London, see pages 1-10); its main advantage being speed and sensitivity. Utilising this assay format simple samples can be analysed in a matter of minutes while complex ones routinely take only half an hour with a detection capability provided down to 10-¹² mol/l ATP. There is however a need for methods which provide still further sensitivity when detecting microorganisms or their contents while retaining speed and ease of performance.

The present inventor has now determined that the speed and sensitivity of this ATP based method can be enhanced significantly by shifting the target of the assay from ATP to the enzyme which generates it, adenylate kinase. Adenylate kinase is an enzyme used by all organisms for the conversion of adenosine diphosphate (ADP) to adenosine triphosphate (ATP). The targeting of this enzyme in preference to ATP, by using the preferred method, apparatus and kits of the invention, allows the detection of down to at least 10⁻²⁰ moles of intracellular marker adenvlate kinase.

It is known to assay adenylate kinase using the luciferase/luciferin system (see Brolin et al Journal of Biochemical and Biophysical Methods 1 (1979) 163-169) for the purpose of determining its activity in certain mammalian and plant tissues (Rodionova et al Fisiologiya Rastenii (1978) 25, 4, P731-734 for plants). The use of such assay system for the detection of microorganisms however has not been suggested and the advantages of doing such, ie. enhanced sensitivity so provided, have not been relevant to those studying the enzyme itself.

Although adenylate kinase is present in smaller quantities than ADP or ATP, its use as a biological marker for microorganisms provides enhanced sensitivity with a typical amplification available of 400,000 by measuring its presence through the ATP it produces; that is for every mole of enzyme present 400,000 moles of ATP are converted to ATP in a 10 minute incubation. Thus estimation of the enzyme by measuring the substrate of product of the reaction it catalyses provides for detection down to as low as 10⁻²⁰ moles.

A first aspect of the present invention provides a method for determining the presence and/or amount of microorganisms and/or their intracellular material present in a sample comprising detecting and/or estimating the amount of adenylate kinase therein using its ability to convert adenosine diphosphate (ADP) to adenosine triphosphate (ATP) and relating that to the presence or amount of microorganisms and/or their intracellular material. This conversion is enabled by adding ADP to samples.

Adenosine triphosphate (ATP) is preferably detected by use of the luciferin/luciferase system to provide a photometrically detectable signal indicative of the amount of ATP in the sample.
Luciferin/luciferase preparations and methods for their use in assaying ATP will be well known to those skilled in the art and are commercially available (eg. see Brolin et al). A typical formulation contains eg. 0.1 to 10mg/litre luciferase, 15 to 1000µmol/litre D-luciferin, and agents such as Mgcl₂, EDTA, BSA, and pH7 buffer (see eg. EP 054676).

As with any amplified assay, the sensitivity of the adenylate kinase assay of the present invention is visited by the purity of the reagents. In this case the significant contaminants are ATP in the ADP substrate and adenylate kinase in the luciferase preparation. For use as a sensitive assay for microorganisms, particularly where these may be potentially harmful and need detecting in low numbers, it is necessary that the purity of each of the reagents be as high as possible with respect to the substance with which it is to react in the assay.

To address the first problem, high purity commercial ADP (>99.5% purity) is preferably used after further purification by column chromatography. This is desirable because even small amounts of contaminating ATP may be sufficient to cause a high background reading. For example, using a diethylaminoethylcellulose column and 0.02mM hydrochloric acid eluent, ATP is eluted more slowly from the column than ADP to a degree enabling substantial separation. Other chromatographic media and eluent combinations may also be used to similar effect. The initial fractions with high ADP to ATP ratios are retained for use. Purity is assessed by luciferin/luciferase reagent bioluminescence after adenylate kinase action to measure ADP levels and without adenylate kinase to measure ATP contaminant levels. A further method for removing ATP from the ADP substrate uses enzymes that specifically degrade ATP, such as luciferase or apyrase. Such enzymes may also be used to further purify chromatographically purified ADP, or alternatively enzymically purified ADP may be treated by column chromatography. It will be noted that apyrase is also an ADPase, but as it is more active on ATP and the ADP is present at much higher levels this does not present a significant problem.

With regard to the second problem adenylate kinase, as an essential "housekeeping" enzyme, is present in all organisms and is generally present in luciferase preparations. It may only be a minor contaminant but since the aim is to measure very low adenylate kinase levels in samples, its presence in the luciferase may be a limiting factor.

The molecular weights of luciferase and adenylate kinase are sufficiently different, being 61kD and 21kD respectively. Furthermore luciferase is relatively hydrophobic, whereas adenylate kinase occurs as a soluble enzyme. It is thus possible to remove adenylate kinase from luciferase preparations by, eg. size exclusion chromatography, reverse phase chromatography, or both. Alternatively or in addition to this, the problem of adenylate kinase contamination of luciferase can be avoided by adding the bioluminescent reagents (luciferase and luciferin) just before or as measurements are taken so that any contaminating adenylate kinase does not have the time to produce a significant effect.

In order to render all the adenylate kinase associated with a target microorganism available to the ADP and luciferase/luciferin assay reagents of the present invention it will be necessary to disrupt them such that intracellular material is released or otherwise exposed to the reagents. Such disruption might be carried out using mechanical means such as an ultrasonic generator, by use of osmotic shock optionally in association with cold shock or such agents as lysozyme or, more conveniently, by use of detergents. Such detergents are commercially available and commonly referred to as 'extractants'. Typical extractants include generic cationic detergents such as CTAB (cetyl trimethyl ammonium bromide), and proprietory agents such as Enzymatics* ATP releasing agent, Biotrace* XM extractant (available from Biotrace, Bridgend UK), and Lumac* NRM (nucleotide releasing agent available from Lumac BV, Holland). When using CTAB a convenient preparation will include 0.01 to 1% CTAB in water. eg 0.2%, but other concentrations may occur to those skilled in the art [*RTM].

Thus before adding ADP and luciferase/luciferin reagent(s) to an assay sample suspected of containing microorganisms it is preferred to disrupt these to render their intracellular contents accessible to luminometry reagents by use of disrupting agent. If it is desired to distinguish between target cells and cells such as those of fungal spores it is possible to run two separate assays treating one with nonionic detergent capable of disrupting only these spores and multi -cellular 'isomatic' animal cells (eg.Triton* TX-100) and the other with cationic detergent 'extractants' detailed above for disrupting all cells. It is possible to carry out these assays on the same sample if an ATPase such as apyrase is added between detergent/luciferase /measurement cycles; one cycle using nonionic and the other cationic detergent in a first cycle step. [*RTM]

The apparatus of the present invention is characterised in that it comprises means for receiving a sample to be analysed for the presence of microorganisms in an aqueous suspension thereof, means for addition of ADP, luciferase and luciferin to the suspension and means for detecting light produced. Preferably the apparatus includes a means for adding detergent to the suspension before the means for adding the luciferase and luciferin. Preferably the ADP is added before the luciferase and luciferin, eg with the detergent, to allow time for the generation of ATP, but all agents may be added together if using glow kinetics. Preferably the luciferase/luciferin reagent(s) is/are added separately from the ADP.

Preferably the apparatus includes a detection means for determining the amount of light emitted from the suspension on addition of the luciferase and luciferin and optionally includes a computer processor and visual display unit for receiving a signal from the detection means indicative of the amount of light emitted and for calculating from that the likely presence and amount of microorganisms and displaying results. Such calculation might be facilitated by programing the processor to take account of a set order of incoming signals, some of which will be controls including blank and nonionic detergent runs, or take account of pre-input standards eg. temperature.

A preferred apparatus will include a conveyor means which receives a volume of liquid medium holding the sample from one or more reagent stations to the light detection means. Thus for example a conveyor receives a series of sample vessels, preferably luminometry vessels, which are preloaded with a phosphate buffer including aqueous liquid suspension of material to be tested for the presence of microorganisms or which are passed through station of the apparatus where such suspension is placed therein. The vessels for example may be open topped and passed thereafter on the conveyor under a detergent adding station, under ADP and luciferase/luciferin adding stations and then through a light detector station. The light detector may be of standard luminometer format eg. Biotrace Multi-lite or Biotrace M3.

Light may be measured by residence of the sample volume, eg. luminometer tube, within the light detector immediately after or simultaneously with addition of the luciferase and luciferin. Thus in a preferred apparatus the luminometry reagents are added just prior entry into or within the light detector station. A preferred apparatus measures the light emitted immediately after reagent addition, then again after a set period of time. Alternatively the light emmited is detected over a suitably long period such that it can be assessed cumulatively eg. where glow kinetics are used.

The test kit of the present invention comprises the essential reagents required for the method of the invention, ie. adenosine diphosphate together with luciferase and luciferin. Preferably the kit includes all these reagents with the luciferase and luciferin being provided as a single reagent solution, with a further detergent in the it reagent suitable for disrupting the target cells for which the assay is intended. Usually for assaying microorganisms only cationic detergent is needed, whereas if fungal spores and somatic cells are likely to be significant then a further nonionic detergent reagent might be included to asess their numbers. The kit is in the form of a single package preferably including instructions as to how to perform the method of the invention; the reagents being provided in containers and being of strength suitable for direct use or after dilution.

A preferred test kit of the invention comprises ADP reagent which is of purity higher than 99.5%, and a luciferase reagent that is substantially free of adenylate kinase activity. Alternatively the luciferase/luciferin ratio used, reflected in the kit instructions for use and/or in their relative concentrations, is such that the luciferase is capable of acting upon the luciferin substrate sufficiently quickly that the luciferase associated adenylate kinase produces ATP after the initial emission measurement is finished.

The preferred purified reagents may be provided by the methods described above. It is noted that adenylate kinase activity in the luciferase may be deleted by leaving the luciferase to stand for a period of months or years.

The methods, apparatus, reagents and kits of the present invention will now be illustrated by way of example with reference to the following non-limiting Examples and Figures. Further embodiments of the invention will occur to those skilled in the art in the light of these.

### FIGURES

Figure 1: shows a graph of increase in luminometer counts per minute for various amounts of E. coli assayed according to Examples 4 and 5.

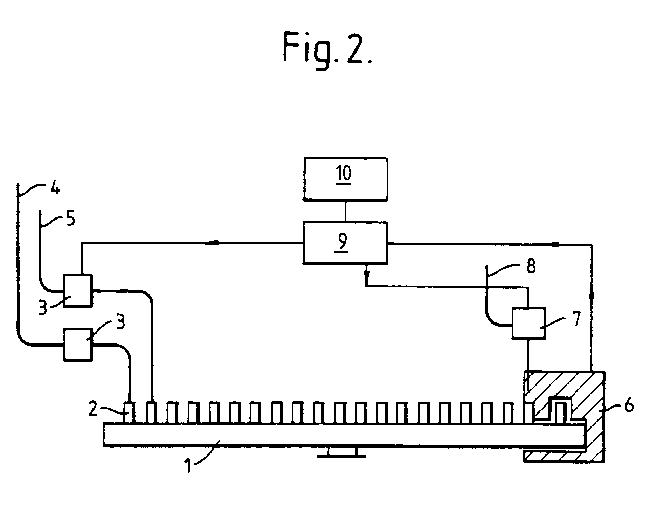

Figure 2: shows a diagrammatic representation of the apparatus of Example 6.

### Example 1: Preparation of purified adenosine diphosphate reagent.

Liquid chromatography was used to further purify commercial high purity (>99.95%) ADP (Sigma). Small columns were made from 10ml disposable plastic syringe bodies and circles of glass fibre filter paper (Whatman GF/A) were placed inside the columns covering their outlets. The chromatographic medium, diethylaminoethylcellulose (Whatman DE-52), was carefully poured into each column and allowed to settle, giving a bed volume of about 4mls. Another glass filter paper circle was placed on top of the column packing.

After washing the column with about 15mls of eluent (0.02M HCl). 100mM high purity ADP in about 0.5mls of O.02M HCl was applied and elution carried out with 0.02M HCl flowing at 1ml per minute. Fractions of 3 to 4mls were collected in disposable plastic cuvettes which allowed the optical density - and thereby the ADP - to be monitored conveniently (at 265nm). The initial fractions, with high ADP:ATP ratios, were retained for use.

To determine the success of this purification. commercially available luciferin/luciferase preparations (Enzymatix, Cambridge, UK and HM by Biotrace. Bridgend, UK) were used according to the manufacturers instructions to detect the amount of ATP present. Similar tests were carried out in the presence of adenylate kinase to determine ADP levels.

50µl of a 1/3,000 dilution of 100mM commercial (Sigma) high purity ADP gave a luminometer count of 8,919, this being a measure of ATP impurity levels. After incubation with 100 femtomoles of adenylate kinase the same sample gave a count of 1,370,839, this being a measure of the quantity of ADP. A column purified fraction derived from this ADP solution gave an ATP count of 223 and an adenylate kinase count of 1,442,054 under the same conditions. The signal to background ratio in this case was improved from 153 to 6466.

### Example 2: Alternative preparation of adenosine diphosphate reagent

High purity ADP was further purified by the action of apyrase on contaminating ATP. 0.1mM solutions were made with ADP derived from two different sources. One (A) was sold as 98% pure and the other (B) as 99% pure. Commercially available luciferin/luciferase preparations were used to determine ATP, the luminometer counts from samples A and B being 54,768 and 305,500 respectively. 8µls of a 100 unit per ml solution of apyrase (potato apyrase: Sigma) were then added to 10mls of 0.1mM solutions of A and B. After incubation at room temperature for about 22 hours followed by boiling to destroy the apyrase. luminometer counts of 5,100 for A and 6,600 for B were obtained showing a marked decrease in the amount of contaminating ATP.

### Example 3: Assessment of assay of free adenylate kinase.

Stock solutions of the adenylate kinase for assay were made in pH 7.2 phosphate buffered saline containing 1% BSA and 0.25% Triton X-100. and the assay was performed in disposable 3ml plastic tubes suitable for luminometry. 200µl of pH 7.8 Tris buffer was pipetted into the assay tube and to this was added 100µl of approximately lmM ADP (purified as detailed above). 10µl of adenylate kinase, diluted in pH 7.8 Tris buffer, was then added to start the reaction. The tube was whirli-mixed and left to incubate at room temperature. After incubation for 10 minutes, 100 to 150µl of luciferin/luciferase reagent was added and the light output from the ATP formed by activity of the adenylate kinase was measured immediately in a luminometer (see Table 1).

It should be noted that the sensitivity of the assay can be increased by using higher concentrations of ADP in the reaction medium as the Kₘ for adenylate kinase is in the millimolar range. Commercially available ADP containing significant amounts of ATP, renders use of such millimolar quantities of ADP undesirable but use of the purified ADP of the invention allows such increase with attendant advantages. Increasing time of incubation likewise will increase sensitivity.

Unknown adenylate kinase levels were estimated by reference to a calibration curve relating known concentrations of adenylate kinase to ATP produced in the 10 minute incubation. Because of the sensitivity of the assay it is desirable to take precautions against accidental contamination by ATP or adenylate kinase. Assays should be carried out in a laminar flow hood using ATP free solutions disposable rubber gloves and low ATP-plastics consumables where possible.

**TABLE 1:**

| relationship between the amount of ATP detected and the amount of adenylate kinase (AK) present: amplification is ATP formed/mole AK in 10 minute incubation. | | |
|---|---|---|
| Moles of AK | pMoles of ATP formed | Amplification |
| 1 femtomole | 322 | 320000 |
| 500 attomoles | 235 | 470000 |
| 250 attomoles | 97.5 | 390000 |
| 125 attomoles | 55.2 | 440000 |
| 62 attomoles | 26.5 | 420000 |
| 31 attomoles | 12.7 | 400000 |
| 16 attomoles | 5.7 | 360000 |
| 8 attomoles | 3.9 | 500000 |
| 4 attomcoles | 2.1 | 540000 |
| 2 attomoles | 0.8 | 410000 |
| 1 attomole | 0.6 | 610000 |
| 0.5 attomole | 0.3 | 610000 |

It will be realised that when adenylate kinase is being used to determine the likely presence of particular organisms accurate quantification may be enhanced if the amount of adenylate kinase they are expected to contain is be estimated. Hence calibration curves made up using the specific target organisms might be best used.

### Example 4: Assay of E. coli.

A one week old E. coli broth culture containing approximately 2.2 x 10⁷ microorganisms per 200µl of phosphate buffered saline pH7.4 was used as stock and diluted in successive dilutions of 10 with that buffer to give a range of samples of from 10⁷ to 0.1 organisms per 200µl sample.

Each 200µl sample was added to a 3ml luminometer tube, 10µl 1mM ADP and 100µl of 0.1% aqueous cetyl trimethyl ammonium bromide added and the resultant mixture incubated at room temperature for 1 minute. On completion of the incubation 100µl aged Biotrace HM (2 years old having no detectable adenylate kinase activity) was added and the light emitted determined over a first 10 second interval and then over 10 second intervals up to one minute to determine the increase in light in cumulative fashion using a Biotrace M3 luminometer. The initial signal value was subtracted from the final reading to gain a measure of the signal in counts per minute.

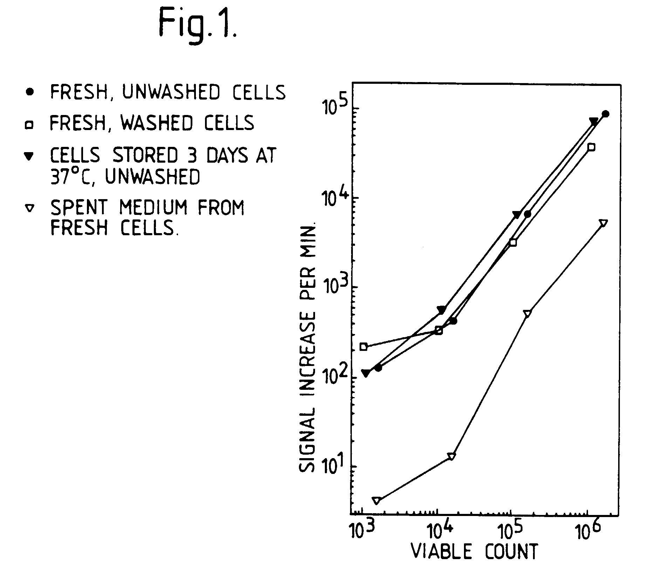

Counts above control obtained over the minute incubation varied with number of E. coli as follows: 10⁶ - 39297cpm; 10⁵ - 3199cpm; 10⁴ - 189cpm; 10³ - 67cpm; 10² - 26cpm. Further results are shown in Fig 1.

The effect of extractant upon the luciferase/luciferin system is known to be important (see eg. Simpson et al (1991) J. Biolumin Chemilumin 6(2) pp97-106); with cationic detergents being known to potentiate the reaction but to cause gradual inactivation of luciferase. anionic detergent inhibiting the reaction and nonionic and zwitterionic detergents being known to potentiate over a wide range. In order to assess the effects of detergent upon the adenylate kinase assay of E. coli cells the protocol used above was altered in so far as different 'extractants' were used to assay 10⁷ E. coli in 200µl of phosphate buffered saline.

The highest counts were obtained using Lumac NRM while CETAB gave 226924 cpm and two other extractants gave 79,280 and 29,280 cpm respectively. This is not surprising in the light of tne Simpson et al. findings regarding the deleterious effects of cationic and anionic detergents on luciferase; it being considered likely that these reagents, designed for use with luciferase/luciferin alone for ATP assay, have inhibitory effect on adenylate kinase.

### Example 5: location of adenylate kinase detected in E. coli assay.

In order to determine the location of the adenylate kinase detected in the assay of Example 4 the number of counts per minute obtained using fresh unwashed E. coli cells, fresh washed cells, cells stored for 3 days at 37°C and unwashed and the medium from fresh cells as the sample. The results from these assays showed that most of the adenylate kinase is intracellular, less than 10% being released into the medium, and that the adenylate kinase levels do not vary significantly with the age of the cells. (see Figure 1)

### Example 6: Apparatus of the invention.

An apparatus of the invention consists of a carousel conveyor (1) mounting racks suitable for holding open topped luminometer tubes (2) with a number of reagent addition stations being placed at various points along its direction of travel. Luminometer tubes (2) containing samples to be assayed are loaded onto the carousel at the start of the run and pass to a first station where computer controlled peristaltic pumps (3) operate supply of cationic detergent (4) and ADP reagent (5) to deliver the required 100µl and 10µl respectively. The carousel run next carries the tube to a luminometer enclosure (6) where simultaneously 100µl luciferase/luciferin reagent (eg. Biotrace HM) is added using a computer controlled peristaltic pump (7) to control delivery from supply (8). The tube takes at least 1 minute to travel from the detergent/ADP station to the luminometer/luciferase /luciferin station to allow microorganism disruption and ATP synthesis.

After addition of the luciferase/luciferin reagent the tube remains in the luminometer enclosure for 70 seconds while 7 readings of counts/10 second period are taken, with the cumulative value after 10 seconds being subtracted from that after 70 seconds to give the counts per minute. This calculation is carried out in an associated computer (9) fed with a cpm signal by the luminometer and results for each tube applied to the carousel displayed on a visual display unit (10). In this fashion the computer is capable of controlling the time of delivery of reagents to a known tube to vary the incubation period if required.

### Example 7: Test kit of the invention.

A test kit of the invention consists of a container holding purified ADP solution (>99.95% pure) prepared as described in Example 1 or 2 at 10mM (increased concentration to that of the method described in Example 4 to increase sensitivity); a container holding aged luciferase/luciferin solution (Biotrace HM) and a container holding cetyl trimethyl ammonium bromide (0.1% in water); all packaged together with instructions as to use in the method of the invention. For use in mobile laboratories the package may be in the form of a plastics box having resilient mountings for each container, ie foam filling with recesses in the shape of the container exterior.

Optionally included in the package is a container of nonionic detergent solution (Triton X-100 0.2% or equivalent) and/or a container holding an ATPase such as apyrase for the destruction of ATP released by the action of the nonionic detergent on a sample rendering it suitable for reassay by addition of the cationic detergent.

Phosphate buffer may be included in the kit as a separate buffer or may be included in the detergent or ADP reagent containers particularly if these are in final concentration. Alternatively the buffer may be included with the detergent and/or ADP in a concentrated form for dilution.

## Claims

1. A method for determining the presence and/or amount of microorganisms and/or their intracellular material present in a sample comprising detecting and/or estimating the amount of adenylate kinase therein using its ability to convert adenosine diphosphate (ADP) to adenosine triphosphate (ATP) and relating that to the presence or amount of microorganisms and/or their intracellular material.

2. A method as claimed in claim 1 wherein the sample is an aqueous suspension or solution and the estimation of adenylate kinase therein is carried out by adding ADP under conditions whereby any adenylate kinase present will convert that to ATP, adding luciferase and luciferin agents, determining the amount of light emitted from the sample and relating that to the presence and amount of adenylate kinase.

3. A method as claimed in claim 2 wherein the ADP has an ADP to ATP ratio of 2000 to 1 or more.

4. A method as claimed in claim 2 wherein the ADP is that obtainable by separation of commercial high purity ADP of over 98% purity into ADP and ATP fractions by passing it through a column of diethyl-aminoethyl cellulose by elution of 0.02M hydrochloric acid at a rate of lml per minute.

5. A method as claimed in claim 2 wherein the ADP has purity with respect to ATP such that 50µl of a 1/3,000 dilution 100mM solution of the ADP gives off less than 1,000 counts per minute when assayed with 100µl Biotrace Enzyme HM luciferase and luciferin reagent.

6. A method as claimed in claim 5 wherein the ADP gives off less than 300 counts per minute when assayed with 100µl Biotrace Enzyme HM luciferase and luciferin agent.

7. A method as claimed in claim 2 wherein the ADP has purity with respect to ATP whereby the molar ratio of ADP to ATP is in excess of 6000.

8. A method as claimed in claim 2 wherein the ADP is obtainable by treating ADP of 98% or more purity with an ATPase for sufficient time to reduce its ATP to 0.1 mole % or less.

9. A method as claimed in claim 2 wherein the luciferase agent has been created to eliminate its adenylate kinase activity.

10. A method as claimed in claim 9 wherein the adenylate kinase in the luciferase has been removed by size exclusion chromatography, reverse phase chromatography, or both.

11. A method as claimed in claim 2 wherein the luciferase and/or luciferin are added just before or as measurements are taken such that any contaminating adenylate kinase does not have the time to produce a significant effect.

12. A method as claimed in any one of the preceding claims wherein the sample is pretreated to disrupt any microorganisms present such that intracellular material is released or otherwise exposed to the assay reagents.

13. A method as claimed in claim 12 wherein the disruption treatment is carried out using detergents.

14. A method as claimed in claim 13 wherein the detergent comprises a cationic detergent.

15. A method as claimed in claim 13 wherein the assay is also carried out on a sample pretreated with nonionic detergent and the amount of ATP produced is subtracted from that produced using the cationic detergent assay.

16. An apparatus for detecting the presence of microorganisms or their intracellular contents by a method as claimed in any one of claims 1 to 15, said apparatus comprising means for receiving a sample to be analysed in the form of an aqueous suspension, means for addition of ADP, luciferase and luciferin to the sample, means for detecting light produced wherein a conveyor is provided for moving the sample and means relative to each other for the purpose of sequential operation.

17. An apparatus as claimed in claim 16 further including a means for adding detergent to the suspension before the means for adding the luciferase and luciferin.

18. An apparatus as claimed in claim 17 wherein the ADP reagent is added with the detergent.

19. An apparatus as claimed in any one of claims 16 to 18 further including a light detecting station where luciferase and luciferin are added to the sample prior to monitoring light emitted therefrom with the means for detecting light.

20. An apparatus as claimed in claims 16 comprising a conveyor means which receives a volume of liquid medium holding the sample and carries it through one or more reagent stations to the light detection means.

21. An apparatus as claimed in claim 20 comprising a conveyor adapted to receive a series of luminometry vessels which are preloaded with an aqueous liquid suspension of material to be tested for the presence of microorganisms or which are passed through a station of the apparatus where such suspension is placed therein.

22. A test kit for the detection and/or quantification of microorganisms by a method as claimed in any one of claims 1 to 15 comprising adenosine diphosphate, luciferase and luciferin.

23. A test kit as claimed in claim 22 further comprising a detergent reagent suitable for disrupting the target microorganism cells for which detection and/or quantification is intended.

24. A test kit as claimed in claim 22 of 23 comprising cationic and nonionic detergent reagents.

25. An ADP reagent for use in the detection and/or quantification of microorganisms or their intracellular contents by a method as claimed in any one of claims 1 to 15 comprising ADP of purity with respect to ATP such that the mole ratio of ADP to ATP is more than 2000 to 1.

26. An ADP reagent comprising ADP as claimed in claim 25 wherein the mole ratio of ADP to ATP is 99.99 to 0.01 or more.

27. A test kit as claimed in any one of claims 22 to 24 wherein the ADP reagent is as claimed in claim 25 or 26.

28. A test kit as claimed in any one of claims 22 to 24 or 27 wherein the luciferase is substantially free of adenylate kinase activity.

29. A test kit as claimed in any one of claims 22 to 24 or 27 wherein the luciferase/luciferin ratio used, or the instructions therein refer to dilution of one or other such reagents, are such that the luciferase is capable of acting upon the luciferin substrate sufficiently quickly such that the luciferase associated adenylate kinase produces ATP after the initial emission measurement is finished.

## Patentansprüche

1. Verfahren für die Bestimmung der Gegenwart von Mikroorganismen und/oder der Menge dieser Mikroorganismen und/oder des intrazellulären Materials dieser Mikroorganismen in einer Probe, das den Nachweis und/oder die Abschätzung der Menge der darin enthaltenen Adenylatkinase unter Verwendung der Fähigkeit der Adenylatkinase, Adenosindiphosphat (ADP) in Adenosintriphosphat (ATP) umzuwandeln, und die Herstellung eines Zusammenhangs zwischen dieser Umwandlung und der Gegenwart oder der Menge der Mikroorganismen und/oder des intrazellulären Materials dieser Mikroorganismen umfaßt.

2. Verfahren nach Anspruch 1, wobei die Probe eine wäßrige Suspension oder eine Lösung ist und die Abschätzung der darin enthaltenen Adenylatkinase durchgeführt wird durch Zugabe von ADP unter Bedingungen, unter denen beliebige vorhandene Adenylatkinase das ADP zu ATP umwandelt, Zugabe von Luciferase und Luciferin, Bestimmung der Menge des von der Probe abgegebenen Lichts und Herstellen eines Zusammenhangs mit der Gegenwart und der Menge der Adenylatkinase.

3. Verfahren nach Anspruch 2, wobei das ADP ein ADP/ATP-Verhältnis von 2000/1 oder darüber aufweist.

4. Verfahren nach Anspruch 2, wobei das ADP das ADP ist, das durch Auftrennen von im Handel erhältlichem ADP hoher Reinheit mit einer Reinheit über 98 % in ADP- und ATP-Fraktionen durch Hindurchlaufenlassen durch eine Diethylaminoethylcellulose-Säule und Eluieren mit 0,02 M Chlorwasserstoffsäure bei einer Geschwindigkeit von 1 ml pro Minute erhältlich ist.

5. Verfahren nach Anspruch 2, wobei das ADP, bezogen auf ATP, eine solche Reinheit aufweist, daß 50 µl einer 1/3000-Verdünnung einer 100-mM-Lösung von ADP eine Zählrate von weniger als 1000 Zählimpulsen pro Minute ergeben, wenn eine Untersuchung mit 100 µl Biotrace Enzyme HM Luciferase- und Luciferinreagens durchgeführt wird.

6. Verfahren nach Anspruch 5, wobei für das ADP weniger als 300 Zählimpulse pro Minute gemessen werden, wenn es mit 100 µl Biotrace Enzyme HM Luciferase- und Luciferinreagens untersucht wird.

7. Verfahren nach Anspruch 2, wobei das ADP, bezogen auf ATP eine Reinheit aufweist, die einem ADP/ATP-Molverhältnis von mehr als 6000 entspricht.

8. Verfahren nach Anspruch 2, wobei das ADP durch Behandlung von ADP mit einer Reinheit von mehr als 98 % mit einer ATPase während eines Zeitraums, der für die Verringerung des ATP-Gehalts auf 0,1 Mol-% oder darunter ausreicht, erhältlich ist.

9. Verfahren nach Anspruch 2, wobei das Luciferase-Reagens zur Entfernung der Adenylatkinase-Aktivität vorbehandelt worden ist.

10. Verfahren nach Anspruch 9, wobei die in der Luciferase enthaltenen Adenylatkinase durch Größen-Ausschluß-Chromatographie, Umkehrphasenchromatographie oder beide Chromatographieverfahren entfernt worden ist.

11. Verfahren nach Anspruch 2, wobei die Luciferase und/oder das Luciferin unmittelbar bevor oder während der Durchführung der Messungen zugegeben werden, so daß für verunreinigende Adenylatkinase keine ausreichende Zeit verbleibt, einen merklichen Effekt hervorzurufen.

12. Verfahren nach einem der vorhergehenden Ansprüche, wobei die Probe zur Zerstörung aller enthaltener Mikroorganismen vorbehandelt wird, so daß das intrazelluläre Material freigesetzt oder in anderer Weise den Untersuchungsreagenzien ausgesetzt wird.

13. Verfahren nach Anspruch 12, wobei die Behandlung zur Zerstörung der Mikroorganismen unter Verwendung von grenzflächenaktiven Stoffen durchgeführt wird.

14. Verfahren nach Anspruch 13, wobei der grenzflächenaktive Stoff einen kationischen grenzflächenaktiven Stoff umfaßt.

15. Verfahren nach Anspruch 13, bei dem die Untersuchung außerdem mit einer Probe durchgeführt wird, die mit einem nichtionischen grenzflächenaktiven Stoff vorbehandelt wurde, und bei dem die Menge des erzeugten ATPs von der Menge ATP abgezogen wird, die bei der Untersuchung unter Verwendung des kationischen grenzflächenaktiven Stoffs erzeugt wird.

16. Vorrichtung für den Nachweis der Gegenwart von Mikroorganismen oder des Zellinhalts dieser Mikroorganismen nach einem Verfahren nach einem der Ansprüche 1 bis 15, wobei die Vorrichtung Mittel zur Aufnahme einer in Form einer wäßrigen Suspension vorliegenden Probe, die analysiert werden soll, Mittel für die Zugabe von ADP, Luciferase und Luciferin zu der Probe, Mittel für den Nachweis von erzeugtem Licht umfaßt, wobei Fördermittel für die Bewegung der Probe und von Einrichtungen relativ zueinander zum Zwecke des sequentiellen Betriebs vorgesehen sind.

17. Vorrichtung nach Anspruch 16, die außerdem Mittel für die Zugabe von grenzflächenaktiven Stoffen zu der Suspension aufweist, die vor den Mitteln für die Zugabe von Luciferase und Luciferin angeordnet sind.

18. Vorrichtung nach Anspruch 17, wobei das ADP-Reagenz mit dem grenzflächenaktiven Stoff zugegeben wird.

19. Vorrichtung nach einem der Ansprüche 16 bis 18, die außerdem eine Station zum Lichtnachweis umfaßt, bei der Luciferase und Luciferin zu der Probe gegeben werden, bevor das von der Probe emittierte Licht mit der Lichtnachweis-Einrichtung gemessen wird.

20. Vorrichtung nach Anspruch 16, die Fördermittel umfaßt, die die Probe halten und ein Volumen flüssiges Medium aufnehmen und die Probe durch eine oder mehrere Stationen für die Zugabe von Reagenzien zu der Lichtnachweis-Einrichtung bewegen.

21. Vorrichtung nach Anspruch 20, die Fördermittel umfaßt, die an die Aufnahme einer Reihe von Luminometer-Gefäßen angepaßt sind, in die vorab eine wäßrige flüssige Suspension eines auf die Gegenwart von Mikroorganismen zu testenden Materials gefüllt wird oder die durch eine Station der Vorrichtung bewegt werden, in der eine derartige Suspension in die Gefäße gegeben wird.

22. Test-Kit für den Nachweis und oder die quantitative Bestimmung von Mikroorganismen nach einem Verfahren nach einem der Ansprüche 1 bis 15, der Adenosindiphosphat, Luciferase und Luciferin enthält.

23. Test-Kit nach Anspruch 22, der außerdem einen grenzflächenaktiven Stoff enthält, der zum Zerstören der Zellen der Zielmikroorganismen, für die der Nachweis und/oder die quantitative Bestimmung vorgesehen ist, geeignet ist.

24. Test-Kit nach einem der Ansprüche 22 und 23, der einen kationischen grenzflächenaktiven Stoff und einen nichtionischen grenzflächenaktiven Stoff als Reagenzien enthält.

25. ADP-Reagens zur Verwendung für den Nachweis und/oder die quantitative Bestimmung von Mikroorganismen oder des Zellinhalts dieser Mikroorganismen nach einem Verfahren nach einem der Ansprüche 1 bis 15, das ADP mit einer solch großen, auf ATP bezogenen Reinheit enthält, daß das ADP/ATP-Molverhältnis größer als 2000/1 ist.

26. ADP-Reagenz, das ADP nach Anspruch 25 enthält, wobei das ADP/ATP-Molverhältnis größer als oder gleich 99,99/0,01 ist.

27. Test-Kit nach einem der Ansprüche 22 bis 24, wobei das ADP-Reagens ein ADP-Reagens nach einem der Ansprüche 25 und 26 ist.

28. Test-Kit nach einem der Ansprüche 22 bis 24 oder 27, wobei die Luciferase im wesentlichen frei von Adenylatkinase-Aktivität ist.

29. Test-Kit nach einem der Ansprüche 22 bis 24 oder 27, wobei das verwendete Luciferase/Luciferin-Verhältnis oder die in dem Kit enthaltene Gebrauchsanleitung, die sich auf die Verdünnung eines oder mehrerer derartiger Reagenzien bezieht, so ist, daß die Luciferase schnell genug auf das Luciferin-Substrat einzuwirken vermag, so daß die mit der Luciferase assoziierte Adenylatkinase ATP erst nach Beendigung der ersten Messung der Lichtemission erzeugt.

## Revendications

1. Méthode de détection de la présence et/ou de la quantité de microorganismes et/ou de leur matière intracellulaire dans un échantillon, comprenant la détection et/ou l'estimation de la quantité d'adénylate kinase qui s'y trouve par l'utilisation de son aptitude à convertir l'adénosine disphosphate (ADP) en adénosine triphosphate (ATP) et sa mise en relation avec la présence ou la quantité de microorganismes et/ou de leur matière intracellulaire.

2. Méthode suivant la revendication 1, dans laquelle l'échantillon est une suspension ou solution aqueuse et l'estimation de l'adénylate kinase qui s'y trouve est effectuée par addition d'ADP dans des conditions dans lesquelles toute adénylate kinase présente le convertit en ATP, addition de luciférine et de luciférase, détermination de la quantité de lumière émise par l'échantillon et mise en relation de cette quantité de lumière avec la présence et la quantité d'adénylate kinase.

3. Méthode suivant la revendication 2, dans laquelle l'ADP a un rapport ADP:ATP de 2000:1 ou plus.

4. Méthode suivant la revendication 2, dans laquelle l'ADP est celui que l'on peut obtenir par séparation d'ADP de haute pureté du commerce, ayant une pureté supérieure à 98%, en fractions d'ADP et d'ATP par passage de cet ADP sur une colonne de diéthylaminoéthyl-cellulose avec élution par l'acide chlorhydrique 0,02M à une vitesse de 1 ml par minute.

5. Méthode suivant la revendication 2, dans laquelle l'ADP a une pureté par rapport à l'ATP telle que 50µl d'une dilution à 1/3000 d'une solution à 100mM de l'ADP délivrent moins de 1000 coups par minute lorsqu'elle est analysée avec 100µl de réactif à la luciférase et à la luciférine Enzyme HM Biotrace.

6. Méthode suivant la revendication 5, dans laquelle l'ADP délivre moins de 300 coups par minute lorsqu'il est analysé avec 100µl de réactif à la luciférase et à la luciférine Enzyme HM Biotrace.

7. Méthode suivant la revendication 2, dans laquelle l'ADP a une pureté par rapport à l'ATP telle que le rapport molaire ADP:ATP dépasse 6000.

8. Méthode suivant la revendication 2, dans laquelle l'ADP peut être obtenu par traitement d'ADP de pureté égale ou supérieure à 98% avec une ATPase pendant une durée suffisante pour réduire sa teneur en ATP à 0,1 mole % ou moins.

9. Méthode suivant la revendication 2, dans laquelle la luciférase a été traitée pour en éliminer l'activité en adénylate kinase.

10. Méthode suivant la revendication 9, dans laquelle l'adénylate kinase présente dans la luciférase a été éliminée par chromatographie d'exclusion stérique, chromatographie à phase inversée, ou les deux.

11. Méthode suivant la revendication 2, dans laquelle la luciférase et/ou la luciférine sont ajoutées juste avant ou au moment de l'exécution des mesures afin que toute adénylate kinase présente comme impureté n'ait pas le temps de produire un effet significatif.

12. Méthode suivant l'une quelconque des revendications précédentes, dans laquelle l'échantillon est prétraité afin de rompre tous microorganismes présents de manière que la matière intracellulaire soit libérée ou autrement exposée aux réactifs analytiques.

13. Méthode suivant la revendication 12, dans laquelle on effectue le traitement de rupture en utilisant des détergents.

14. Méthode suivant la revendication 13, dans laquelle le détergent comprend un détergent cationique.

15. Méthode suivant la revendication 13, dans laquelle l'analyse est aussi effectuée sur un échantillon prétraité avec un détergent non ionique et la quantité d'ATP produite est soustraite de la quantité produite en utilisant l'analyse au détergent cationique.

16. Appareil de détection de la présence de microorganismes ou de leur contenu intracellulaire par une méthode suivant l'une quelconque des revendications 1 à 15, cet appareil comprenant des moyens permettant de recevoir un échantillon à analyser sous la forme d'une suspension aqueuse, des moyens d'addition d'ADP, de luciférase et de luciférine à l'échantillon, des moyens de détection de la lumière produite, un convoyeur étant prévu pour le déplacement de l'échantillon et des moyens les uns par rapport aux autres en vue d'une opération en série.

17. Appareil suivant la revendication 16, comprenant en outre un moyen d'addition de détergent à la suspension en amont du moyen d'addition de la luciférase et de la luciférine.

18. Appareil suivant la revendication 17, dans lequel le réactif ADP est ajouté avec le détergent.

19. Appareil suivant l'une quelconque des revendications 16 à 18, comprenant en outre un poste de détection de lumière où de la luciférase et de la luciférine sont ajoutées à l'échantillon avant le contrôle de la lumière émise par l'échantillon avec le moyen de détection de la lumière.

20. Appareil suivant la revendication 16, comprenant un moyen convoyeur qui reçoit un volume de milieu liquide contenant l'échantillon et qui le transporte vers le moyen de détection de la lumière en le faisant passer par un ou plusieurs postes à réactif.

21. Appareil suivant la revendication 20, comprenant un convoyeur conçu pour recevoir une série de récipients de luminométrie qui sont préalablement chargés d'une suspension liquide aqueuse de matière à tester pour détecter la présence de microorganismes ou qui sont amenés à passer par un poste de l'appareil où cette suspension y est introduite.

22. Kit analytique pour la détection et/ou la numération de microorganismes par une méthode suivant l'une quelconque des revendications 1 à 15, comprenant de l'adénosine diphosphate, de la luciférase et de la luciférine.

23. Kit analytique suivant la revendication 22, comprenant en outre un réactif détergent qui convient pour rompre les cellules cibles de microorganismes faisant l'objet de la détection et/ou de la numération.

24. Kit analytique suivant la revendication 22 ou 23, comprenant des réactifs détergents cationique et non ionique.

25. Réactif à l'ADP destiné à être utilisé dans la détection et/ou la numération de microorganismes ou au dosage quantitatif de leur contenu intracellulaire par une méthode suivant l'une quelconque des revendications 1 à 15, comprenant de l'ADP de pureté telle, par rapport à l'ATP, que le rapport molaire de l'ADP à l'ATP soit supérieur à 2000:1.

26. Réactif à l'ADP comprenant de l'ADP selon la revendication 25, dans lequel le rapport molaire de l'ADP à l'ATP est égal ou supérieur à 99,99:0,01.

27. Kit analytique suivant l'une quelconque des revendications 22 à 24, contenant le réactif à l'ADP suivant la revendication 25 ou 26.

28. Kit analytique suivant l'une quelconque des revendications 22 à 24 ou 27, dans lequel la luciférase est sensiblement dépourvue d'activité en adénylate kinase.

29. Kit analytique suivant l'une quelconque des revendications 22 à 24 ou 27, dans lequel le rapport luciférase/luciférine utilisé ou les indications qui y sont données en ce qui concerne la dilution de l'un ou l'autre de ces réactifs, sont tels que la luciférase soit capable d'agir sur le substrat de luciférine avec une rapidité suffisante pour que l'adénylate kinase associée à la luciférase produise de l'ATP après que la mesure de l'émission initiale est terminée.
